# EUROPEAN PATENT APPLICATION

(11) **EP 4 534 007 A1**
(43) Date of publication of application: **09.04.2025**
(21) Application number: 23811671.9
(22) Date of filing: 15.05.2023
(51) Int. Cl.: A61B 5/11, A44B 99/00, A61B 5/113

(54) **ATTACHMENT TOOL**

(30) Priority: 26.05.2022 JP 2022086043; 26.05.2022 JP 2022086045; 27.07.2022 JP 2022119550; 26.10.2022 JP 2022171537
(71) Applicant: D.O.N Co., Ltd., Nagoya-shi, Aichi 453-0804 (JP)
(72) Inventor: IESAKI, Takashi, Nagoya-shi, Aichi 453-0804 (JP)
(74) Representative: IK-IP LTD
(86) International application number: PCT/JP2023/018126
(87) International publication number: WO 2023/228797

(57) **Abstract**

[Problem] An object of the present invention is to provide an attachment fixture that facilitates appropriate mounting of a physical information acquisition device that performs sensing inside a user's body.

[Solution] The present invention to solve the above problems is an attachment fixture 1 that can attach a physical information acquisition device 2 that measures physical information of a user by emitting a measurement wave including a millimeter wave or a microwave from an emission and entrance surface 22 toward the user to a belt 3, the attachment fixture including: an attachment fixture body 11(Q); a mounting portion that can be mounted to a waist part of the user; and an attachment portion 12 to which the physical information acquisition device 2 can be attached.

## Description

### Technical Field

The present invention relates to an apparatus for attaching a device for acquiring physical information, in particular information on a user's breathing.

### Background Art

A physical information acquisition device automatically measures and records a pulse, a body surface temperature, a blood oxygen level, or the like of the user by being mounted, and performs health management of the user and formation of big data.

As an example of the physical information acquisition device, there are a wristwatch-type device that is wrapped around the user's arm as described in Patent Literature 1, in which a photoplethysmography or an optical sensor is provided on a back surface to measure the blood oxygen level and the like of the user, and a device that is mounted on the user's head to measure brain waves, line of sight, or the like as described in Patent Literature 2.

By the way, among the physical information, data on organs is very useful for identifying diseases of respiratory system and circulatory system, and the like.

As a device for acquiring respiratory data, Patent Literature 3 describes a sensor in which sensors are attached to two portions of a seat belt, a distance between both the sensors is measured by radio waves emitted from each sensor, and body motion is measured from the distance to detect the user's breathing and the like. Furthermore, Patent Literatures 4 to 7 describe various attachment fixtures for attaching a physical information acquisition device, and Patent Literatures 8 to 10 describe various attachment fixtures for attaching an article to a belt or the like.

### Citation List

### Patent Literature

Patent Literature 1: JP 2016-533846 A
Patent Literature 2: JP 3217017 U
Patent Literature 3: JP 2018-201581 A
Patent Literature 4: WO 2020/017627 A
Patent Literature 5: JP 2009-503734 A
Patent Literature 6: JP 2018-500643 A
Patent Literature 7: US 2015/0,164,422 A
Patent Literature 8: WO 2009/141942 A
Patent Literature 9: JP 3076482 U
Patent Literature 10: JP H09-299492 A

### Summary of Invention

### Technical Problem

By the way, by irradiating an object to be measured with a measurement wave such as a standing wave radar and measuring the reflected wave by various sensors, the distance between the object to be measured and the sensor can be directly observed.

Then, by setting the object to be measured by the measurement wave as an organ such as the lung or the intestinal tract or the body surface of the user, data related to breathing, eating, and the like can be acquired by measuring the body motion.

On the other hand, in the inventions described in the cited literatures, accurate data cannot be acquired because a position of the organ or the like to be measured fluctuates greatly in relation to the position of the sensor.

In view of the above problems, an object of the present invention is to provide an attachment fixture for facilitating appropriate attachment and detachment of a physical information acquisition device that performs sensing of a body part of a user.

### Solution to Problem

The present invention to solve the above problems is an attachment fixture that can attach a physical information acquisition device that measures physical information of a user by emitting a measurement wave including a millimeter wave or a microwave from an emission and entrance surface toward the user to a belt, the attachment fixture including: an attachment fixture body; a mounting portion that can be mounted to a waist part of the user; and an attachment portion to which the physical information acquisition device can be attached.

With such a configuration, the physical information acquisition device can be attached at an appropriate position for sensing inside the body using the measurement wave. With an attachment to the waist part, the position of the physical information device relative to the user's body is fixed regardless of a movement of the user, and a body motion measurement using the measurement wave can be appropriately performed.

In a preferred mode of the present invention, the mounting portion is provided with an insertion portion through which the belt is inserted, and further includes at least one of a second insertion portion or an adjustment portion that adjusts a length of the belt at a distance from the insertion portion.

With such a configuration, the attachment fixture can be mounted to the belt wrapped around the waist part, and the attachment of the physical information acquisition device can be performed more comfortably.

In a preferred mode of the present invention, the physical information acquisition device includes an attached portion that can be attached to the attachment portion.

With such a configuration, the user can easily recognize an attachment position, and can stably maintain an attachment state.

In a preferred mode of the present invention, the belt includes an insertion portion to be inserted into the attached portion at an end portion, and the attached portion includes an insertion port into which the insertion portion is inserted.

With such a configuration, interference between the measurement wave emitted from the physical information acquisition device and other components is difficult to occur, and appropriate data on breathing can be obtained, while removal of the physical information acquisition device can be facilitated by a simple operation of the user.

In a preferred mode of the present invention, the physical information acquisition device has a plurality of planes including the emission and entrance surface, and the insertion port opens in one plane adjacent to the emission and entrance surface.

With such a configuration, interference between the emission and entrance surface and the belt is difficult to occur, and removal of the physical information acquisition device can be facilitated.

In a preferred mode of the present invention, the attached portion is provided to face each other on two surfaces different from the emission and entrance surface.

With such a configuration, interference between the measurement wave emitted from the physical information acquisition device and other components is difficult to occur, and appropriate data on breathing can be obtained, while a holding force of the physical information acquisition device by the attachment fixture can be enhanced.

In a preferred mode of the present invention, the attachment fixture body includes a transmission portion that transmits the measurement wave between the insertion portion and the second insertion portion or the adjustment portion.

With such a configuration, interference between the measurement wave emitted from the physical information acquisition device and other components is difficult to occur, and appropriate data on breathing and the like can be obtained.

In a preferred mode of the present invention, the adjustment portion is provided with a hole insertion portion that penetrates the belt.

With such a configuration, the position of the attachment fixture relative to the belt can be easily fixed.

In a preferred mode of the present invention, the attachment portion is provided with a sliding portion that slides with the attached portion.

With such a configuration, removal of the physical information acquisition device can be facilitated by a simple operation of the user.

In a preferred mode of the present invention, the attachment fixture body is a frame shape that leaves a central portion.

With such a configuration, the user can touch the physical information acquisition device in a state of being attached to the attachment fixture, so that removal can be facilitated.

In a preferred mode of the present invention, the attachment portion includes a protrusion portion that protrudes from the attachment fixture body.

With such a configuration, it is easy for the user to check the position of the attachment portion, so that a sliding operation can be easily performed, and the holding force of the physical information acquisition device can be further improved.

In a preferred mode of the present invention, the attachment portion includes a holding portion that holds an attached state in which the physical information acquisition device is attached.

With such a configuration, the holding force of the attached physical information acquisition device can be easily improved.

In a preferred mode of the present invention, the attachment fixture body is provided to be separable.

With such a configuration, the measurement wave emitted by the physical information acquisition device can be directly emitted to the object to be measured without being disturbed, and measurement accuracy can be improved, and weight reduction can be easily realized.

In a preferred mode of the present invention, the mounting portion includes a clip portion that can be mounted by sandwiching a member,
with such a configuration, the physical information acquisition device can be more easily attached to the waist part of the user.

In a preferred mode of the present invention, the attachment portion is provided with a lateral sliding attachment portion to which the physical information acquisition device is attached by sliding in a horizontal direction.

With such a configuration, removal of the physical information acquisition device can be facilitated by a simple operation of the user, and detachment due to gravity becomes difficult to occur.

In a preferred mode of the present invention, the attachment portion is provided with a vertical sliding attachment portion to which the physical information acquisition device is attached by sliding in a vertical direction.

With such a configuration, removal of the physical information acquisition device can be facilitated by a simple operation of the user, and the position of the attachment portion can be easily visualized during use.

In a preferred mode of the present invention, the attachment portion is configured such that a distance from a portion mounted to the physical information acquisition device to the mounting portion is longer than a distance from the attached portion to the emission and entrance surface in a direction orthogonal to the emission and entrance surface.

With such a configuration, the physical information acquisition device and the belt do not interfere with each other, and the user can easily attach the physical information acquisition device without applying excessive force.

Further, a preferred mode of the present invention is a waist belt having the attachment fixture.

With such a configuration, the physical information acquisition device can be more stably attached to the waist part of the user.

Further, the present invention is an attachment method for attaching a physical information acquisition device that measures physical information of a user by emitting a measurement wave including a millimeter wave or a microwave from an emission and entrance surface toward the user to a belt, the attachment method including attaching the physical information acquisition device such that the emission and entrance surface faces the user's body via an attachment fixture that includes an attachment fixture body, a mounting portion that can be mounted to a waist part of the user, and an attachment portion to which the physical information acquisition device can be attached.

By such a method, the physical information acquisition device can be attached to the waist part of the user in an appropriate form for sensing inside the body using the measurement wave.

Further, the present invention is a physical information acquisition device that measures physical information of a user by emitting a measurement wave including a millimeter wave or a microwave from an emission and entrance surface toward the user, the physical information acquisition device being attachable to a belt by an attachment fixture that includes an attachment fixture body, a mounting portion that can be mounted to a waist part of the user, and an attachment portion to which the physical information acquisition device can be attached.

With such a configuration, the physical information acquisition device can be attached to the waist portion of the user in an appropriate form for sensing inside the body using the measurement wave.

### Advantageous Effects of Invention

The present invention to solve the above problem can provide an attachment fixture that facilitates appropriate mounting of a physical information acquisition device that performs sensing inside a user's body.

### Brief Description of Drawings

Fig. 1 is a perspective view of an attachment fixture according to a first embodiment of the present invention.
Fig. 2 is an explanatory drawing of use of the attachment fixture according to the first embodiment of the present invention.
Fig. 3 is an explanatory drawing of use of the attachment fixture according to the first embodiment of the present invention.
Fig. 4 is a view of the attachment fixture according to the first embodiment of the present invention as viewed from various surfaces.
Fig. 5 is a perspective view of an attachment fixture according to a second embodiment of the present invention.
Fig. 6 is an explanatory drawing of use of the attachment fixture according to the second embodiment of the present invention.
Fig. 7 is a view of the attachment fixture according to the second embodiment of the present invention as viewed from various surfaces.
Fig. 8 is a perspective view and an explanatory drawing of an attachment fixture according to a third embodiment of the present invention.
Fig. 9 is a perspective view of an attachment fixture according to a fourth embodiment of the present invention.
Fig. 10 is an explanatory drawing of use of the attachment fixture according to the fourth embodiment of the present invention.
Fig. 11 is a perspective view of an attachment fixture according to a fifth embodiment of the present invention.
Fig. 12 is an explanatory drawing of use of the attachment fixture according to the fifth embodiment of the present invention.
Fig. 13 is a perspective view of an attachment fixture according to a sixth embodiment of the present invention.
Fig. 14 is an explanatory drawing of use of the attachment fixture according to the sixth embodiment of the present invention.
Fig. 15 is an explanatory drawing of an attachment fixture according to a seventh embodiment of the present invention.
Fig. 16 is an explanatory drawing of an attachment fixture according to an eighth embodiment of the present invention.

### Description of Embodiments

Hereinafter, the attachment fixture according to each embodiment of the present invention will be described with reference to the drawings. The description will be given in detail in an order of a configuration of the embodiment, an implementation method, and other examples.

Note that, the embodiments shown below are examples of the present invention, and the present invention is not limited to each of the following embodiments. Further, each part of each embodiment shown below may be combined to form a practical product.

### <<First Embodiment>>

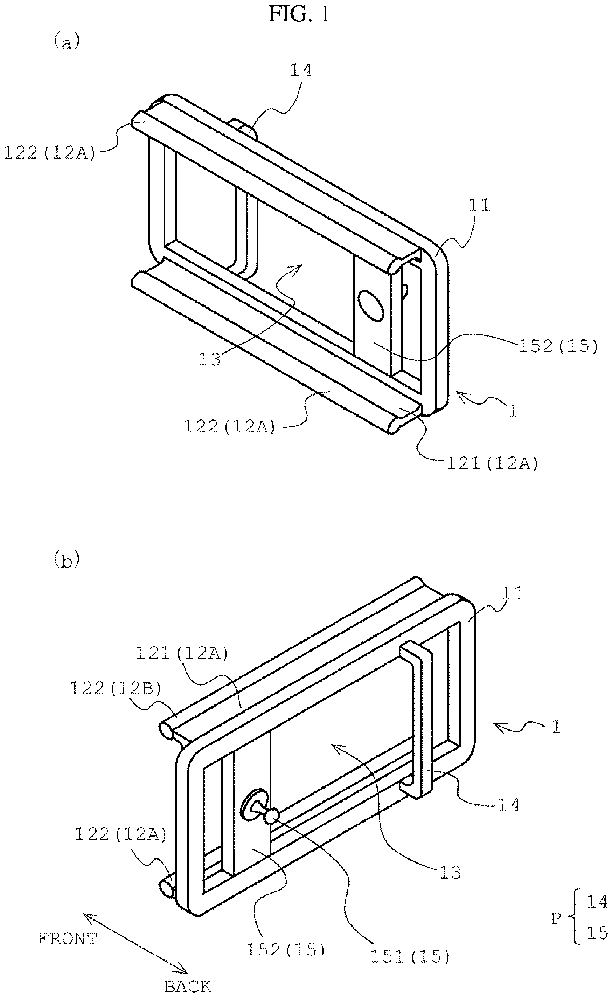

Hereinafter, an attachment fixture 1 according to a first embodiment of the present invention will be described in detail with reference to Figs. 1 to 4.

In the attachment fixture 1 of the present embodiment, a physical information acquisition device 2 is attached by sliding in the horizontal direction on a front surface thereof, and a belt 3 wrapped around a waist part of a user is attached on a back surface thereof. Note that, the horizontal direction mentioned herein is not limited to a completely horizontal direction, and may vary by about 5° depending on an attaching position of the user.

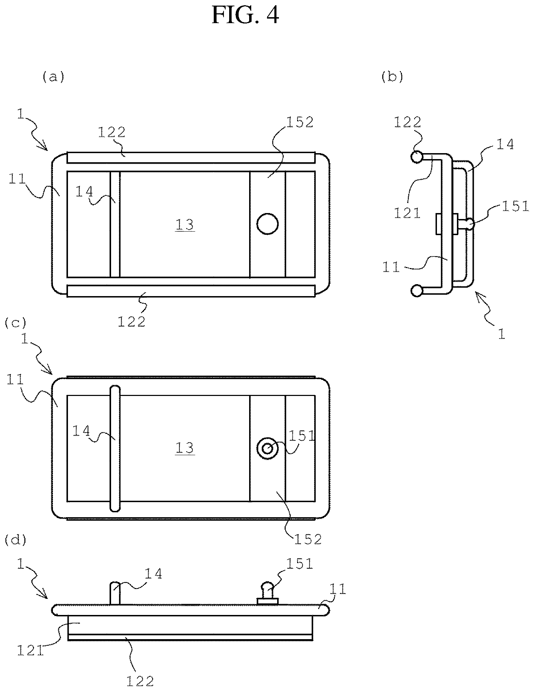

Here, Fig. 1(a) is a perspective view facing the front surface, and Fig. 1(b) is a perspective view facing the rear surface. Fig. 4 represents views of the attachment fixture 1 as viewed from various surfaces, in which Fig. 4(a) is a front view, Fig. 4(b) is a side view, Fig. 4(c) is a rear view, and Fig. 4(d) is a bottom view.

The attachment fixture 1 includes an attachment fixture body 11 serving as a base portion of the attachment fixture 1, an attachment portion 12 to which the physical information acquisition device 2 is attached, a transmission portion 13 that transmits a measurement wave emitted from the physical information acquisition device 2, an insertion portion 14 that has a function as a mounting portion P mounted to the waist part of the user and is attached by inserting the belt 3, and an adjustment portion 15 that can adjust the length of the belt 3.

The attachment fixture body 11 is a member provided to be a substantially rectangular flat plate with rounded corners as a whole, and is made of a metal member such as stainless steel or steel or a plastic member such as polyethylene.

Further, a width of the attachment fixture body 11 is provided to be at least larger than the width of the belt 3, and a horizontal length thereof is provided to be longer than the length of the physical information acquisition device 2. This enables proper attachment.

Note that, considering that it is mounted to the waist part of the user, it may be provided to be curved toward the back surface.

The attachment portion 12 is a member provided to be integrally formed with the attachment fixture body 11, and the physical information acquisition device 2 is attached thereto. The attachment portion 12 includes a protrusion portion 121 provided to protrude from the attachment fixture body 11, and a fitting body 122 in which the physical information acquisition device 2 is fitted and attached at a tip portion of the protrusion portion 121.

In the present embodiment, the attachment portion 12 is provided as a lateral sliding attachment portion 12A to which the physical information acquisition device 2 can be attached by sliding in a lateral direction. More specifically, the attachment portion 12 is formed along each end portion of an upper edge and a lower edge of the attachment fixture body 11 over substantially an entire region excluding rounded corner portions of the edges. Note that, the attachment portion 12 may be provided by dividing along the edges.

The protrusion portion 121 is a member in a substantially flat plate shape provided to protrude from the surface of the attachment fixture body 11. A protrusion length of the protrusion portion is set to be at least longer than the distance from an emission and entrance surface 22 to an attached portion 23, which will be described later, so that the physical information acquisition device 2 does not interfere with the attachment fixture body 11. Preferably, the length is provided to be substantially the same as the distance, so that the distance between the emission and entrance surface 22 and the user's body can be reduced as much as possible.

The fitting body 122 is a component provided at a tip of the protrusion portion 121. A shape of the fitting body is provided to be substantially the same as the shape of the attached portion 23 so that it can fit with the attached portion 23, and is provided to have a portion at least wider than the width of the protrusion portion 121.

In the present embodiment, the fitting body 122 is a cylindrical member having an outer diameter larger than the width of the attached portion 23, but the shape is not limited thereto, and for example, it may be formed in a prismatic shape in which a corner portion is attached to the tip of the protrusion portion 121.

The transmission portion 13 is a region formed in a central portion of the attachment fixture body 11, and transmits a measurement wave such as a sound wave or a radio wave emitted from the emission and entrance surface 22 or a measurement wave such as a sound wave or a radio wave reflected and returned to the emission and entrance surface 22.

In the present embodiment, the transmission portion 13 is provided as a hole in the attachment fixture body 11 to form a hollow region, and thus the attachment fixture body 11 becomes a frame body having a gap. Note that, any configuration may be used as long as the measurement wave can be transmitted, for example, in a case where the measurement wave is a sound wave such as a Doppler wave, a mesh-like fiber member may be used, and in a case where the measurement wave is a radio wave such as a millimeter wave or a microwave, any member such as plastic or wood may be used as long as a member that shields the radio wave such as metal or liquid is not used.

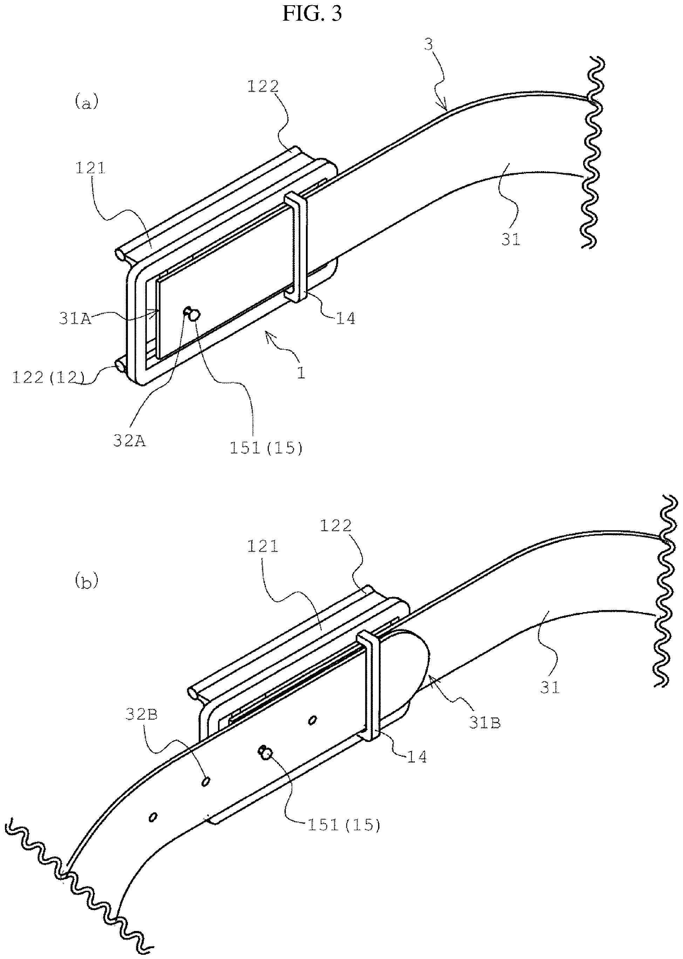

As shown in Fig. 3, the insertion portion 14 is a portion used for attaching the belt 3, and is configured such that the belt 3 can be inserted inside the insertion portion.

In the present embodiment, the insertion portion is configured such that strength can be maintained by being formed as a member that bridges the upper edge and the lower edge of the attachment fixture body 11 provided as a frame body, and the belt 3 can be easily inserted by being provided to protrude in a back side direction and have a U-shape. A protruding height is preferably substantially the same as or slightly larger than twice a thickness of the belt 3.

The adjustment portion 15 is a member capable of adjusting the length of the belt 3, and includes a hole insertion portion 151 for attachment to an attachment hole 32 of the belt 3 to be described later, and an adjustment plate body 152 for holding the hole insertion portion 151 at a predetermined position.

The hole insertion portion 151 is provided substantially at the center of the adjustment plate body 152, and protrudes toward the back surface of the attachment fixture body 11.

Further, the hole insertion portion 151 includes a base portion attached to the adjustment plate body 152, a neck portion protruding from the base portion, and a head portion provided at an end portion of the neck portion. A protruding length of the neck portion is provided to be at least longer than twice the thickness of the belt 3. Further, a diameter of the head portion is configured to be larger than the diameter of the neck portion, and the shape thereof is preferably a spherical or elliptical shape.

The adjustment plate body 152 is a member in a flat plate shape provided to bridge the upper edge and the lower edge of the attachment fixture body 11, which is a frame body, and is provided such that the thickness thereof is substantially the same as the thickness of the attachment fixture body 11.

Further, the base portion of the hole insertion portion 151 is formed at the center portion, and is supported by being sandwiched between the back surface and the front surface.

Note that, the insertion portion 14 and the adjustment portion 15 are provided to be out of the region of the transmission portion 13 that is the center of the frame body, and the transmission portion 13 occupies at least 1/2 of one surface of the attachment fixture body 11.

The physical information acquisition device 2 is a device for acquiring physical information, and includes an acquisition device body 21 having a substantially rectangular parallelepiped shape, the emission and entrance surface 22 that emits and receives a predetermined measurement wave, and the attached portion 23 to which the attachment portion 12 is attached, and includes inside a control unit that controls and processes at least emission and reception of the measurement wave, a recording unit that records data, and a transmission unit that transmits and receives the recorded data to and from the outside.

The acquisition device body 21 is a device in a substantially rectangular parallelepiped shape having a size enough to fit in the palm of a hand with a length of about 4 cm to 9 cm, a width of about 3 cm to 6 cm, and a width of about 0.5 cm to 3 cm, and is provided to be slightly larger than the width of the attachment fixture 1.

Further, a button for turning on a power source and a display unit capable of displaying information related to the physical information acquisition device 2 such as a remaining battery level are provided on a surface on which the attached portion 23 is not provided or a surface adjacent to a surface on which the attached portion 23 is provided.

The emission and entrance surface 22 has a function of emitting a measurement wave (a standing wave of sound waves or radio waves (in particular millimeter waves, microwaves)) toward a predetermined angle and a function as a sensor that receives the measurement wave reflected on the user's body, and a portion where the measurement wave is actually exchanged is set as an emission and entrance position. As a result, the distance between the object to be measured and the emission and entrance surface 22 is grasped.

In the present invention, in order to sense a body part (in particular, the lung, intestinal tract, etc.) particularly in a state of being attached to the waist part of the user, a measurement wave can be emitted upward by about 30° to 70°.

The function of receiving the measurement wave is preferably provided at at least two positions of the emission and entrance surface 22, and thus the movement of the lung can be stereoscopically grasped.

Note that, the surface that outputs the measurement wave and the surface that emits the measurement wave may be different from each other, and a body temperature or the like may also be measured.

The attached portion 23 is a hole portion that is provided near upper and lower end portions of the attachment fixture body 11 and penetrates from one end portion toward the other end portion in the lateral direction. The shape of the attached portion is provided corresponding to the shape of the attachment portion 12, so that it can be attached to the attachment portion 12 protruding from the attachment fixture body 11.

Here, by providing the fitting body 122, movement in a front-rear direction can be restricted and fixed.

Further, the attached portions 23 are provided at intervals corresponding to two attachment portions 12, and with such a configuration, the acquisition device body 21 can be attached even when it is turned upside down.

The belt 3 is a belt-shaped member to be mounted to the waist part of the user, and includes a belt body 31 that is a body portion of the belt, and the attachment hole 32 that is a hole portion for penetrating the adjustment portion 15.

The belt body 31 is a belt-shaped member made of leather or synthetic resin, and has a length that can be wrapped around the user's waist. Hereinafter, the end portions of the belt body 31 will be described as one end portion 31A and the other end portion 31B. One end portion 31A is rounded at the corners.

The attachment hole 32 is a hole portion provided to be attached to the adjustment portion 15, and includes one first attachment hole 32A provided on a side of the one end portion 31A of the belt body 31 and three to five second attachment holes 32B provided near the other end portion 31B at a predetermined interval.

Further, a size of the attachment hole 32 is provided to be larger than the diameter of the neck portion of the hole insertion portion 151 and to be smaller than the diameter of the head portion.

Hereinafter, an implementation method of the present invention will be described in detail with reference to Figs. 1 to 4. The present invention is implemented by a user having a purpose of attaching the physical information acquisition device 2 to his/her waist part. Further, the implementation method shown below is an example, and the implementation method is not limited thereto, and the order may be out of sequence.

### <<Implementation Method>>

First, the user attaches the attachment fixture 1 and the belt 3 to his/her waist.

As shown in Fig. 3(a), the user passes one end portion 31A of the belt body 31 through the insertion portion 14, and then pushes the first attachment hole 32A provided in one end portion 31A into the hole insertion portion 151. Thereafter, the user wraps the belt around his/her waist part while holding an other end side, and inserts the other end portion 31B into the insertion portion 14. Next, as shown in Fig. 3(b), the user inserts the hole insertion portion 151 into the second attachment hole 32B located at a position suitable for the size of his/her waist, and adjusts the length around the belt 3.

Next, the user attaches the physical information acquisition device 2 to the attachment fixture 1.

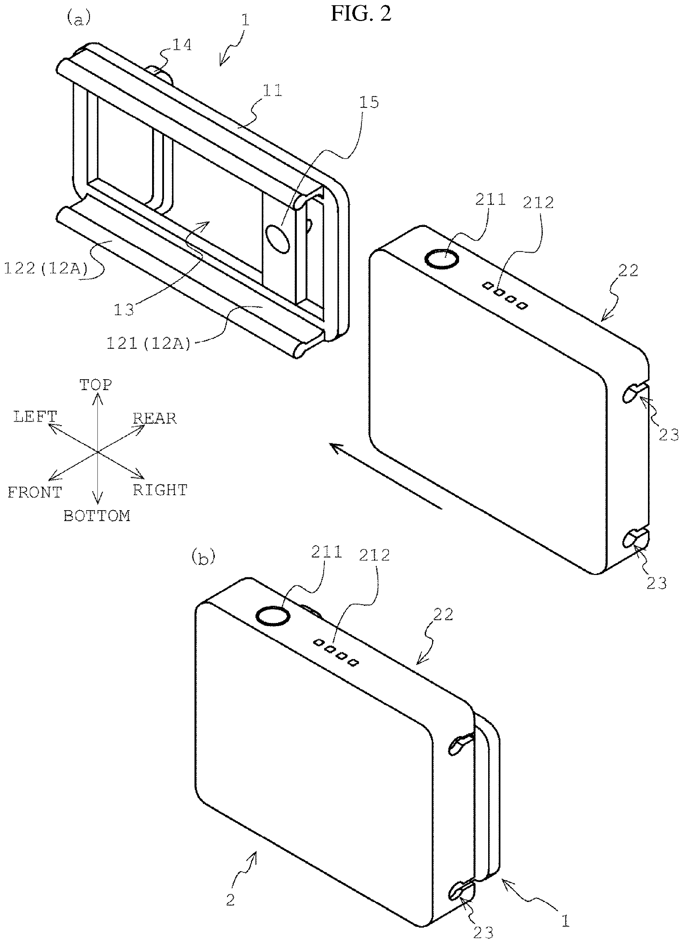

In other words, the user attaches the physical information acquisition device 2 as shown in Fig. 2(b) by sliding it in a lateral direction in a state where the positions of the attachment portion 12 and the attached portion 23 are aligned with each other, as shown in Fig. 2(a).

As described above, the emission and entrance position of the emission and entrance surface 22 overlaps the position of the transmission portion 13, and the portion can be fixed at the waist part, so that the user can appropriately mount the physical information acquisition device 2 that senses the body.

Further, the physical information acquisition device 2 may be provided with a protrusion portion such as the attachment portion 12, and the attachment fixture 1 may be provided with a recess such as an attached portion. Further, a U-shaped member such as the insertion portion 14 may be provided on one surface of the physical information acquisition device 2, and an attachment fixture or a belt may be penetrated and fixed inside the U-shaped member, preferably, two U-shaped members are provided to be parallel to each other, and the attachment fixture 1 and the like can be penetrated simultaneously in each portion to be easily attached.

### <<Second Embodiment>>

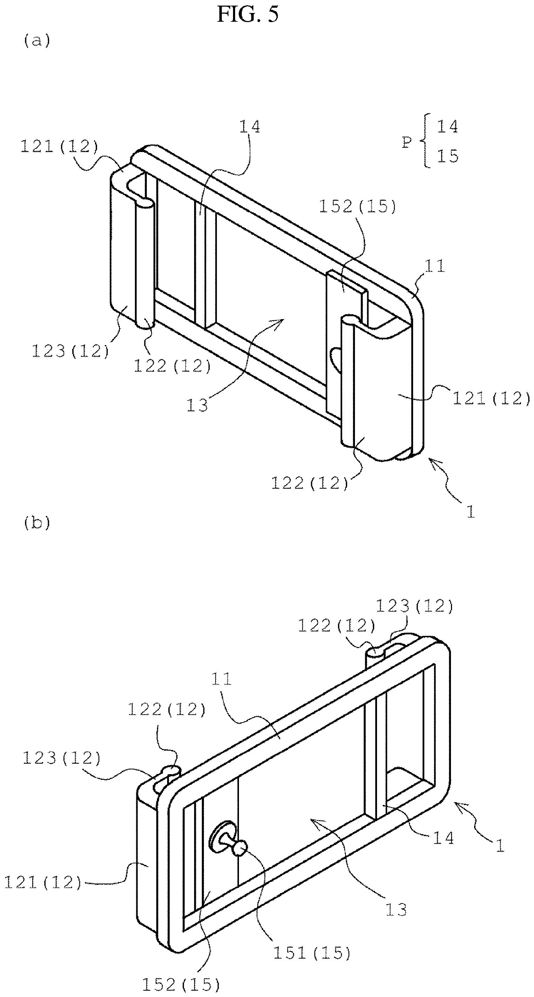

Hereinafter, an invention according to a second embodiment of the present invention will be described in detail with reference to Figs. 5 to 7. The same components as those of the first embodiment are denoted by the same reference numerals, and the description thereof will be omitted. Note that, a vertical direction mentioned herein is not limited to a completely vertical direction, and may vary by about 5° depending on the attaching position of the user.

In the attachment fixture 1 of the present embodiment, the physical information acquisition device 2 is attached by sliding in the vertical direction on the front surface thereof, and the belt 3 wrapped around the waist part of the user is attached on the back surface thereof.

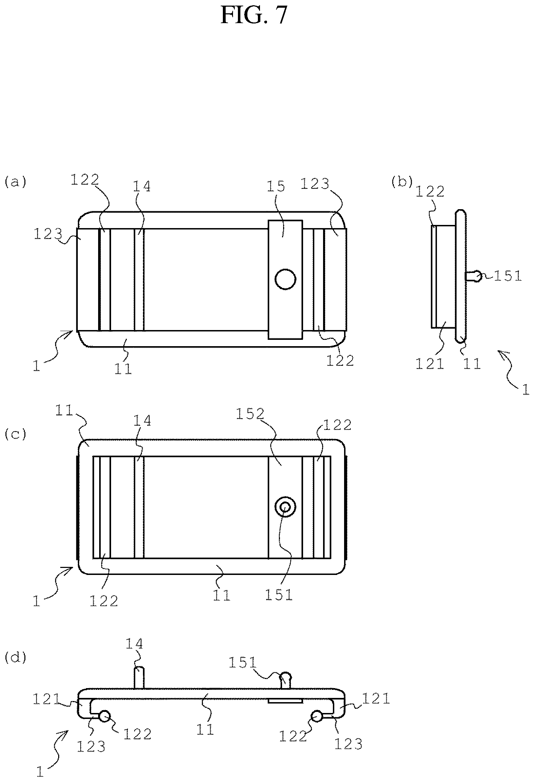

Here, Fig. 7 represents views of the attachment fixture 1 as viewed from various surfaces, in which Fig. 7(a) is a front view, Fig. 7(b) is a side view, Fig. 7(c) is a rear view, and Fig. 7(d) is a bottom view.

As in the first embodiment, the attachment fixture 1 includes the attachment fixture body 11 serving as the base portion of the attachment fixture 1, the attachment portion 12 to which the physical information acquisition device 2 is attached, the transmission portion 13 that transmits a measurement wave emitted from the physical information acquisition device 2, the insertion portion 14 that is attached as the mounting portion P by inserting the belt 3, and the adjustment portion 15 that can adjust the length of the belt 3.

The attachment portion 12 includes the protrusion portion 121 provided to protrude from the attachment fixture body 11, the fitting body 122 in which the physical information acquisition device 2 is fitted at the tip portion of the protrusion portion 121, and a parallel piece 123 that connects the tip portion of the protrusion portion 121 and the fitting body 122.

The attachment portion 12 in the present embodiment is provided as a vertical sliding attachment portion 12B to which the physical information acquisition device 2 can be attached by sliding in a vertical direction. More specifically, the attachment portion 12 is formed along the end portions of two side surface edges of the attachment fixture body 11 over substantially the entire region excluding rounded corner portions of the edge. Note that, the attachment portion 12 may be provided by dividing along the edges.

The protrusion portion 121 in the present embodiment is provided to protrude longer by at least the thickness of the adjustment plate body 152 (or the thickness of the belt body 31) than the distance from the emission and entrance surface 22 to the attached portion 23. Further, the thickness is at least 2 mm or more, and is preferably provided over the entire region from an outer end to an inner end of a frame side surface portion of the attachment fixture body 11.

The fitting body 122 is integrally formed at the end portion of the parallel piece 123, and is provided such that the shape thereof can be fitted to the attached portion 23 and is a shape substantially the same as the shape of the attached portion 23, and is provided to have a portion at least wider than the width of the protrusion portion 121.

The parallel piece 123 is a component that connects a protruding end of the protrusion portion 121 and the fitting body 122, and is a piece that extends from the protrusion portion 121 in a direction toward the center of the attachment fixture body 11. Preferably, it is provided to be parallel to the upper edge and the lower edge of the attachment fixture body 11.

As shown in Fig. 3, the insertion portion 14 is a portion used for attaching the belt 3, and is configured such that the belt 3 can be inserted inside the insertion portion.

In the present embodiment, the insertion portion 14 is provided to bridge the upper edge and the lower edge of the attachment fixture body 11 on the same plane as the attachment fixture body 11, thereby increasing the strength of the attachment fixture body. Preferably, the length of the insertion portion 14 is substantially the same as the length of the belt body 31.

The adjustment portion 15 is a member capable of adjusting the length of the belt 3, and includes a hole insertion portion 151 for attachment to an attachment hole 32 of the belt 3 to be described later, and an adjustment plate body 152 for holding the hole insertion portion 151 at a predetermined position. The adjustment portion 15 is provided to be separated from the side surface edge of the attachment fixture body 11.

The adjustment plate body 152 is a member on a flat plate provided to bridge the upper edge and the lower edge of the attachment fixture body 11, which is a frame body, and is provided such that the thickness thereof is substantially the same as the thickness of the belt body 31 or about 1 mm to 2 mm thicker than the thickness of the belt body 31. In the present embodiment, the length of the adjustment plate body 152 is provided to be substantially the same as a height of the attachment fixture body 11, and is held so as to be attached to a front side of the attachment fixture body 11.

The physical information acquisition device 2 is a device for acquiring physical information, and includes the acquisition device body 21 having a substantially rectangular parallelepiped shape, the emission and entrance surface 22 that emits and receives a predetermined measurement wave, and the attached portion 23 to which the attachment portion 12 is attached.

Preferably, the height thereof is provided to be longer than that of the attachment portion 12, and more preferably, is provided to be longer than that of the attachment fixture body 11.

The attached portion 23 is a hole portion provided near both side surface end portions of the attachment fixture body 11 and provided from the lower end portion toward the upper end portion in the vertical direction. The attached portion 23 is provided to be inserted into and attachable to the fitting body 122 and the parallel piece 123 and to have a shape corresponding to these components.

Preferably, the attached portion 23 is provided to have a height substantially the same as that of the attachment portion 12, and upper ends of the fitting body 122 and the parallel piece 123 are in contact with each other at the lower end portion of the attached portion 23, which is a hole portion, and are fixedly attached here.

Hereinafter, an implementation method of the present invention will be described in detail with reference to Figs. 5 to 7. The present invention is implemented by a user having a purpose of attaching the physical information acquisition device 2 to his/her waist part. Further, the implementation method shown below is an example, and the implementation method is not limited thereto, and the order may be out of sequence.

### <<Implementation Method>>

First, the user attaches the attachment fixture 1 and the belt 3 to his/her waist.

The user inserts one end portion 31A of the belt body 31 into the insertion portion 14 so that the belt has a mountain shape toward the front of the attachment fixture body 11. Thereafter, the first attachment hole 32A is pushed into the hole insertion portion 151. Next, the user wraps the belt around his/her waist part while holding the other end side, and inserts the other end portion 31B between one end portion 31A and the insertion portion 14. Next, as shown in Fig. 3(b), the user inserts the hole insertion portion 151 into the second attachment hole 32B located at a position suitable for the size of his/her waist, and adjusts the length around the belt 3.

At this time, since the insertion portion 14 and the adjustment portion 15 hardly protrude from the side of the user's body, discomfort during mounting can be reduced.

Next, the user attaches the attachment fixture 1 to the physical information acquisition device 2.

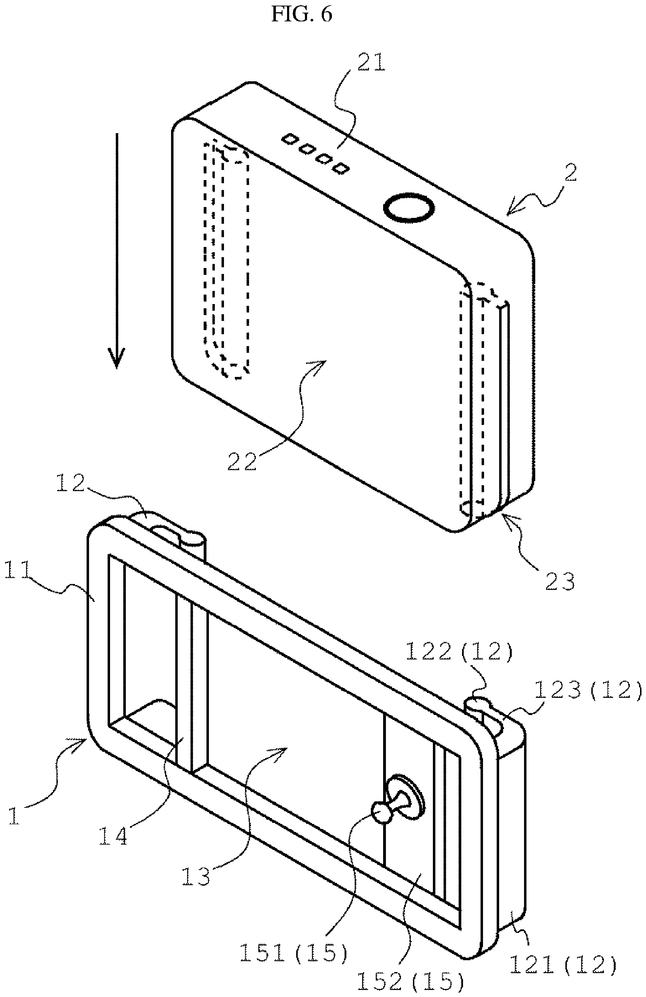

In other words, as shown in Fig. 6, the user attaches the physical information acquisition device 2 by sliding it from top to bottom in a state where the positions of the attachment portion 12 and the attached portion 23 are aligned with each other.

At this time, the emission and entrance position of the emission and entrance surface 22 overlaps with the position of the transmission portion 13.

At this time, even when the attachment fixture 1 is mounted on the user, the user can check the shape of the attachment portion 12 by looking down, so that the physical information acquisition device 2 can be easily attached. Further, the attachment is stabilized by the gravity of the physical information acquisition device itself.

As described above, the emission and entrance position of the emission and entrance surface 22 overlaps the position of the transmission portion 13, and the portion can be fixed at the waist part, so that the user can appropriately mount the physical information acquisition device 2 that senses the body.

Note that, since the attached portion 23 is provided at the end portion of the emission and entrance surface 22, the attachment portion 12 may be configured to simply protrude as in the first embodiment. Further, the parallel piece 123 may be provided to be bent in a U- shape, and the attached portion 23 may be provided on a surface facing the emission and entrance surface 22 correspondingly.

Further, as a modification, the attached portion 23 may be provided to penetrate in the vertical direction.

In this case, the distance between the attachment portions 12 is preferably provided to be longer than the distance between the attached portions 23.

The user warps the attachment fixture body 11 in a front side direction to adjust the distance between the attachment portions 12 to be the same as the distance between the attached portions 23, and attaches the physical information acquisition device 2 in that state.

With such a configuration, a tensile force is applied to the acquisition device body 21 by an elastic force of the attachment fixture body 11, so that the physical information acquisition device 2 can be held. Further, since it is possible to attach the attachment fixture 1 from top or from below, handling performance is improved.

Further, contrary to the above, the attachment fixture body 11 may be provided such that the distance between the attachment portions 12 is shorter than the distance between the attached portions 23, and so that it can be warped in a back side direction. In this case, holding performance of the attachment portion 12 is improved by pressing action of the attachment fixture 1.

### <<Third Embodiment>>

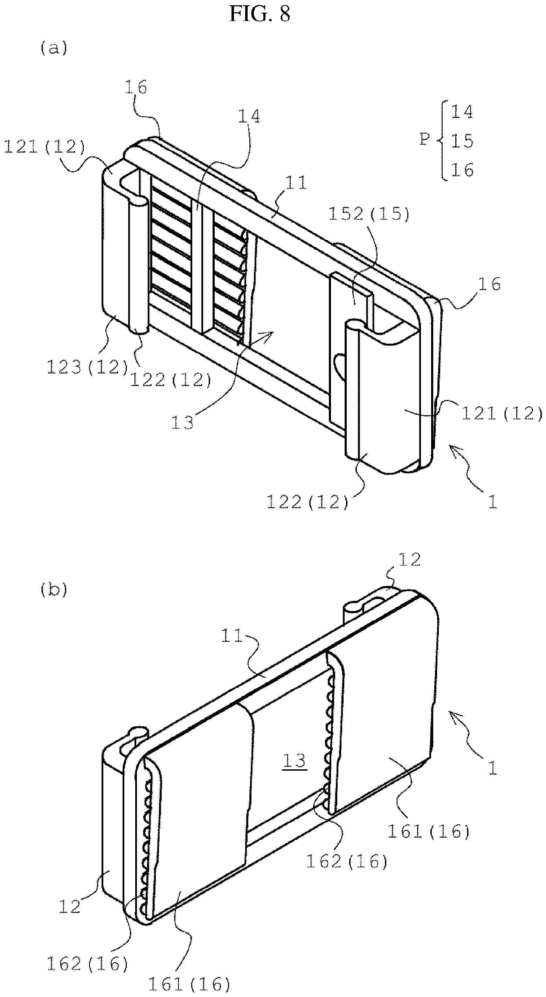

Hereinafter, the attachment fixture 1 according to a third embodiment of the present invention will be described in detail with reference to Fig. 8. The same components as those of the embodiments described above are denoted by the same reference numerals, and the description thereof will be omitted.

The attachment fixture 1 of the present embodiment is provided with the attachment portion 12 to which the physical information acquisition device 2 is attached on the front surface thereof and the mounting portion P that can be mounted on the back surface thereof at the waist part of the user.

The attachment fixture 1 includes the attachment fixture body 11 serving as a base portion of the attachment fixture 1, the attachment portion 12 to which the physical information acquisition device 2 is attached, the transmission portion 13 that transmits a measurement wave emitted from the physical information acquisition device 2, and a clip portion 16 that is provided with the insertion portion 14 and the adjustment portion 15, as the mounting portion P, and is attached by sandwiching the user's clothing (for example, trousers or a skirt).

Note that, the attachment portion 12 may be the lateral sliding attachment portion 12A or the vertical sliding attachment portion 12B.

The clip portion 16 includes a clip body 161 that sandwiches the user's clothing with the attachment fixture body 11, and a friction portion 162 that acts as an anti-slip portion between the clothing and the attachment fixture body 11.

As shown in Fig. 8(b), the clip body 161 is a plate-shaped member provided to extend to an edge of a lower end with the edge of an upper end of the attachment fixture body 11 as a base portion, and two clip bodies are provided at a distance so as to form a gap, and a portion therebetween functions as the transmission portion 13. The transmission portion 13 is configured such that the emission and entrance surface 22 is positioned when the physical information acquisition device 2 is appropriately attached to the attachment portion 12.

The clip body 161 is provided to have a position and size overlapping at least a side edge and the lower edge of the attachment fixture body 11, and is sandwiched on the user's clothing with the side edge and the lower edge of the attachment fixture body 11. Furthermore, in the present embodiment, the clip body 161 is provided with a width to overlap the adjustment plate body 152, and other clip body 161 is provided with a width at a position overlapping the insertion portion 14, so that the attachment is further strengthened by being sandwiched between these members and the clip body 161.

The thickness of the clip body 161 is formed to gradually decrease from the base portion toward the tip portion, so that the clip body 161 near the tip portion is easily deformed to facilitate attachment to clothing.

Further, the clip body 161 protrudes outward at the base portion thereof, and is provided to approach the attachment fixture body 11 as it moves downward and to be substantially in contact with the attachment fixture body at the tip portion, thereby enabling firm attachment in which the members are sandwiched.

In the present embodiment, the friction portion 162 is provided with a plurality of irregularities on a surface in contact with the attachment fixture body 11, and holds the attachment fixture body 11 by sandwiching the clothing on the surface.

On the other hand, the method of generating friction is not limited thereto, and a structure in which a rubber plate, a silicon plate, or the like is pasted may be used.

### <<Implementation Method>>

A method of attaching the attachment fixture 1 according to the present embodiment to the user's clothing is as follows.

The user inserts the upper end of his/her clothing such as trousers, a skirt, or a belt, between the lower end of the clip body 161 and the attachment fixture body 11. As a result, the user can attach the attachment fixture 1 to the waist part.

As described above, the emission and entrance position of the emission and entrance surface 22 overlaps the position of the transmission portion 13, and the portion can be fixed at the waist part, so that the user can appropriately mount the physical information acquisition device 2 that senses the body.

Note that, in the present embodiment, since the insertion portion 14 and the adjustment portion 15 for attaching the belt 3 are provided at a lower part of the clip portion 16, the clip portion can also be mounted through the belt 3. It is more preferable because the handling performance is improved, and in addition to the holding force by the clip portion 16, a holding force is generated by being attached to the belt 3.

### <<Fourth Embodiment>>

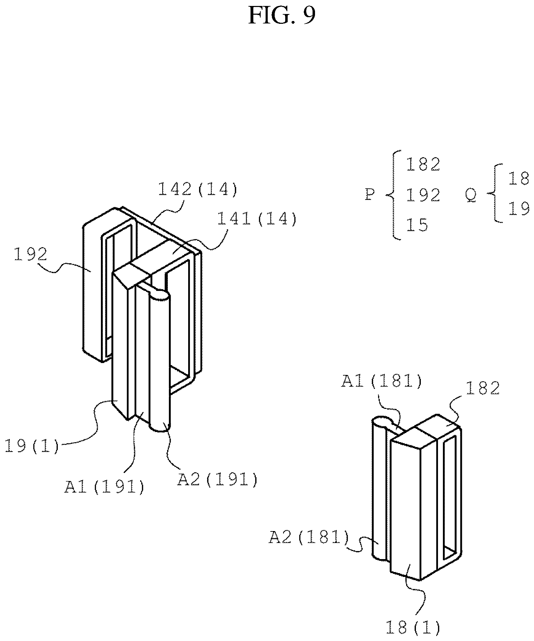

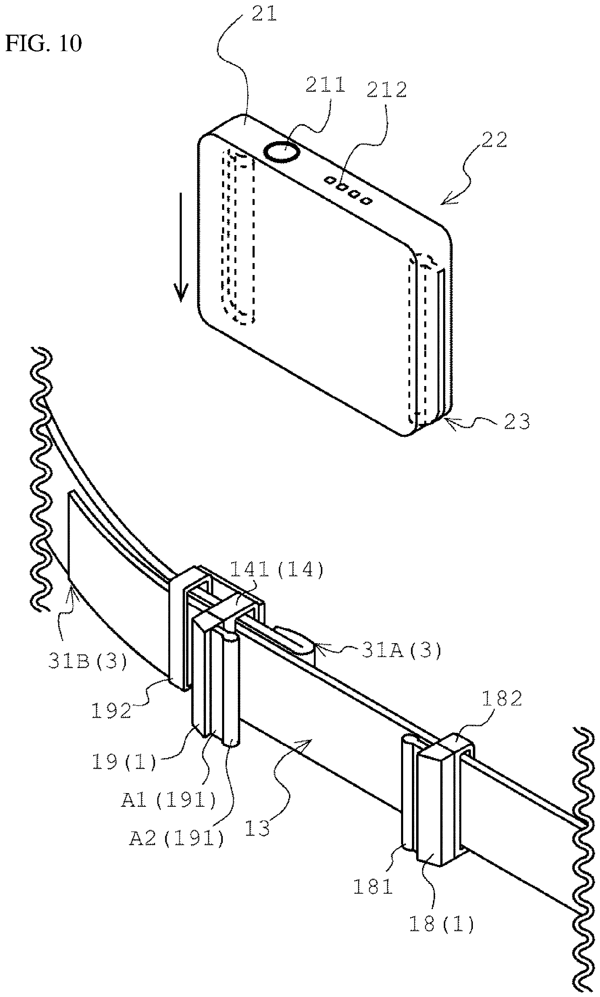

Hereinafter, the attachment fixture 1 according to a fourth embodiment of the present invention will be described in detail with reference to Figs. 9 and 10. The contents common to each embodiment described above are denoted by the same reference numerals and the description thereof will be omitted.

Further, in the following embodiments, since the attachment fixture 1 is separated into a plurality of pieces or has a configuration that can be separated, the plurality of attachment fixtures is collectively referred to as an attachment fixture body Q. This attachment fixture body Q is in the same position as the attachment fixture body 11 in each of the previous embodiments.

The attachment fixture 1 includes a first attachment fixture 18 and a second attachment fixture 19 as the attachment fixture body Q that functions as a body of the attachment fixture. Each attachment fixture 1 includes an attachment portion for attaching the physical information acquisition device 2 and the mounting portion P that is mounted to the waist part of the user, respectively.

The attachment fixture 1 is made of a metal member such as stainless steel or steel, or a plastic member such as polyethylene.

Further, the width of the attachment fixture body Q is provided to be at least larger than the width of the belt 3, and may be provided to be curved toward the back surface in consideration of being mounted to the waist part of the user.

The first attachment fixture 18 includes a body portion having a prismatic shape, a first attachment portion 181 for attaching one surface of the physical information acquisition device 2, and a first insertion portion 182 for passing through the belt 3.

The first attachment portion 181 is provided such that the physical information acquisition device 2 can be attached by sliding it in the vertical direction. More specifically, the first attachment fixture 18 is formed from an upper end to a lower end of the first attachment fixture 18. Furthermore, a protrusion piece A1 that is provided to protrude from the first attachment fixture 18, and a fitting piece A2 in which the physical information acquisition device 2 is fitted at the tip portion of the protrusion piece A1 are included.

The protrusion piece A1 is an edge provided to protrude from the first attachment fixture 18 by 3 mm to 10 mm, and is provided to be parallel to the belt 3 when appropriately attached. The thickness thereof is at least 2 mm or more, and is preferably provided over the entire region from the upper end to the lower end of the attachment fixture body Q.

The fitting piece A2 is a component provided at the tip of the protrusion piece A1. The shape thereof is provided to be substantially the same as the shape of the attached portion 23 to be described later, so that it can be fitted with the attached portion 23, and is provided to have a portion at least wider than the width of the protrusion piece A1.

In the present embodiment, the fitting piece A2 is a cylindrical member having an outer diameter larger than the width of the attached portion 23, but the shape is not limited thereto, and for example, it may be formed in a prismatic shape in which a corner portion is attached to the tip of the protrusion piece A1. Further, by making the size and shape of the fitting pieces A2 facing each other different, the emission and entrance surface 22 to be described later may always face the body.

The first insertion portion 182 is a portion that is integrally molded with the first attachment fixture 18, through which the belt 3 is inserted, and an inserted portion is provided to have a rectangular parallelepiped shape having an opening in a substantially rectangular shape. The size of the opening is provided such that the height is at least larger than the width of the belt 3 and the width is at least larger than the thickness of the belt 3.

The second attachment fixture 19 includes a second attachment portion 191 for attaching one surface of the physical information acquisition device 2, a second insertion portion 192 for passing through the belt 3, and the adjustment portion 15 for adjusting the length of the belt 3.

The structure of the second attachment portion 191 is substantially the same as the structure of the first attachment portion 181, but the width of the opening is provided to be at least larger than twice the thickness of the belt 3, and the second attachment portion is provided to be bilaterally symmetrical with the first attachment portion so as to face each other when attached to the belt 3.

Further, the second insertion portion 192 has substantially the same structure as the first insertion portion 182, but is provided integrally with an adjustment portion body 141.

In the present embodiment, the transmission portion 13 is a gap portion between the first attachment fixture 18 and the second attachment fixture 19. As a result, when the mounting portion P is not mounted and the physical information acquisition device 2 is attached to the attachment portion, the emission and entrance surface is exposed.

Note that, each of the attachment fixtures 1 may be provided to be connectable to each other by a member that transmits a measurement wave. In other words, the transmission portion 13 transmits a measurement wave such as a sound wave or a radio wave emitted from the emission and entrance surface 22, or a measurement wave such as a sound wave or a radio wave reflected and returned to the emission and entrance surface 22. Any configuration may be used as long as the measurement wave can be transmitted, for example, in a case where the measurement wave is a sound wave such as a Doppler wave, a mesh-like fiber member may be used, and in a case where the measurement wave is a radio wave such as a millimeter wave or a microwave, any member such as plastic or wood may be used as long as a member that shields the radio wave such as metal or liquid is not used.

The adjustment portion 15 is integrally molded with the second attachment fixture 19, and is a member having substantially the same shape as the second insertion portion 192 provided in the second attachment fixture 19.

Further, the openings of the first insertion portion 182, the second insertion portion 192, and the adjustment portion 15 are provided such that they face the same direction and are separated from each other at a certain distance. In the present embodiment, a coupling plate 142 is pasted and provided on the surface of the second insertion portion 192 and the surface of the adjustment portion body 141 facing the surface where the second attachment fixture 19 is provided, so that each opening is held in the above state.

The physical information acquisition device 2 is a device for acquiring physical information, and includes the acquisition device body 21 having a substantially rectangular parallelepiped shape, the emission and entrance surface 22 that emits and receives a predetermined measurement wave, and the attached portion 23 to which each attachment portion is attached, and includes inside a control unit that controls and processes at least emission and reception of the measurement wave, a recording unit that records data, and a transmission unit that can transmit and receive the recorded data to and from the outside.

The attached portion 23 is a non-penetrating hole portion provided from the lower end portion toward the upper end portion of the attachment fixture body **Q.** The shape of the hole portion is provided to be substantially the same or slightly larger than the shape corresponding to the shape of each attachment portion, so that the hole portion can be attached to each attachment portion protruding from the attachment fixture body Q.

Here, since the fitting piece A2 is provided, the movement in the direction of falling off can be restricted and fixed. Further, by not penetrating, falling off due to gravity can be prevented.

Further, the distance from both the attachment portions to an opening inner peripheral surface of the insertion portion and the distance from both the attachment portions to the opening inner peripheral surface of the adjustment portion 15 are provided to be at least larger than the distance from the emission and entrance surface 22 to the attached portion 23.

Furthermore, since the emission and entrance surface 22 is provided to face each other on a surface different from the surface on which the attached portion 23 is provided, the physical information acquisition device 2 is attached such that the emission and entrance surface 22 faces the user's body.

The belt 3 is a belt-shaped member to be mounted to the waist part of the user, and includes a belt body 31 that is a body portion of the belt, and the attachment hole 32 that is a hole portion for penetrating the adjustment portion 15.

The belt body 31 is a belt-shaped member made of leather or synthetic resin, and has a length that can be wrapped around the user's waist. Hereinafter, the end portions of the belt body 31 will be described as one end portion 31A and the other end portion 31B.

One end portion 31A of the belt body 31 is made thicker than other portions of the belt body 31 by folding or attaching a metal fitting or the like.

Hereinafter, an implementation method of the present invention will be described in detail with reference to Figs. 9 to 10. The present invention is implemented by a user having a purpose of attaching the physical information acquisition device 2 to his/her waist part. Further, the implementation method shown below is an example, and the implementation method is not limited thereto, and the order may be out of sequence.

### <<Implementation Method>>

First, the user attaches the attachment fixture 1 and the belt 3 to his/her waist.

The user causes the belt body 31 to pass from the other end portion 31B into the adjustment portion 15 and the second insertion portion 192 of the second attachment fixture 19, directly wraps around the waist, and causes it to pass into the first insertion portion 182 of the first attachment fixture 18. Thereafter, the user causes the other end portion 31B to pass into the adjustment portion 15 and the second insertion portion 192 of the second attachment fixture 19. Thus, the first attachment portion 181 and the second attachment portion 191 face each other.

Further, the user adjusts the length of the belt body 31. The belt body 31 can adjust the length of the belt 3 by moving the position of the second attachment fixture 19 to adjust a relative position of one end portion 31A.

0 0 9 61 Next, the user attaches the physical information acquisition device 2 to the attachment fixture 1.

In other words, the user adjusts the distance between the first attachment portion 181 and the second attachment portion 191 to the distance between the attached portions 23, and as shown in Fig. 2, attaches the physical information acquisition device 2 by sliding it from top to bottom in a state where the positions of the first attachment portion 181 and the second attachment portion 191 are aligned with the positions of the attached portions 23.

At this time, since the portion between the first attachment fixture 18 and the second attachment fixture 19 becomes a gap (the transmission portion 13), the emission and entrance position of the emission and entrance surface 22 overlaps with the position of the transmission portion 13, and the portion can be fixed at the waist part, so that the user can appropriately mount the physical information acquisition device 2 that senses the body. Further, it can be easily removed by performing a reverse operation.

### <<Fifth Embodiment>>

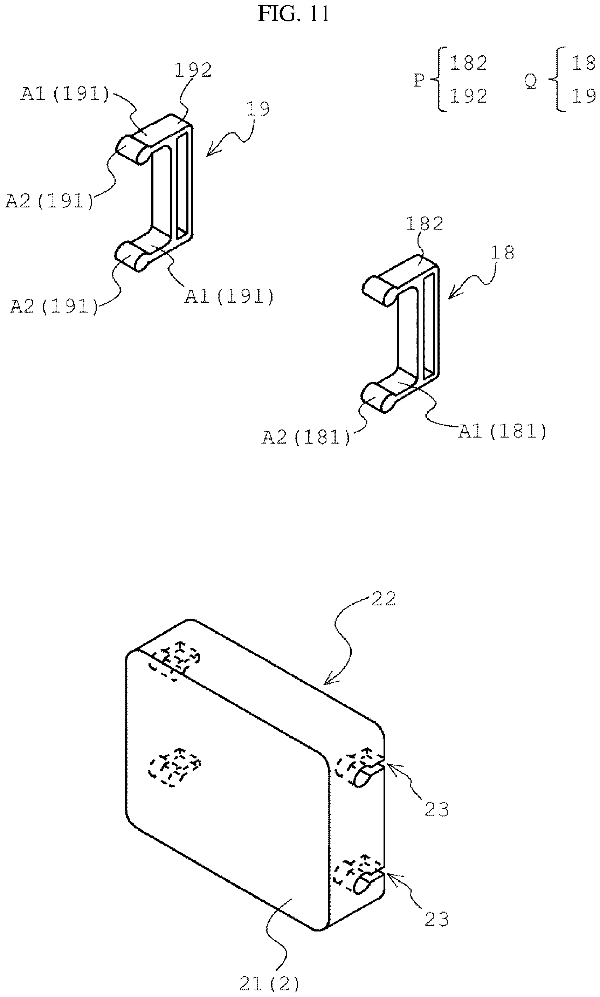

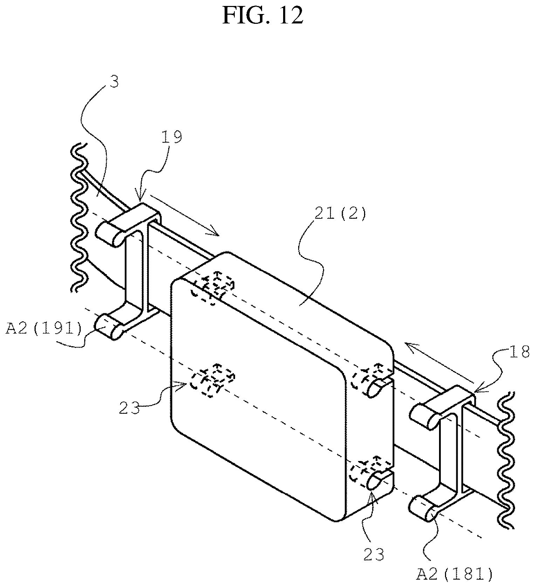

Hereinafter, an invention according to a fifth embodiment of the present invention will be described with reference to Figs. 11 and 12. The same components as those of each embodiment described above are denoted by the same reference numerals, and the description thereof will be omitted.

The attachment fixture 1 includes the first attachment fixture 18 and the second attachment fixture 19. Each attachment fixture 1 includes an attachment portion for attaching the physical information acquisition device 2 and the mounting portion P that is mounted to the waist part of the user, respectively.

The first attachment fixture 18 includes the first attachment portion 181 for attaching one surface of the physical information acquisition device 2, and the first insertion portion 182 for passing through the belt 3.

The first attachment portion 181 is provided such that the physical information acquisition device 2 can be attached by sliding it in the lateral direction, and in the present embodiment, is provided such that the top, bottom, left, and right sides are symmetrical in a front view. More specifically, the first attachment portion 181 is provided at the upper and lower end portions of the attachment fixture 1, respectively. Then, the first attachment portion 181 includes the protrusion piece A1 provided to protrude from the attachment fixture body Q, and the fitting piece A2 into which the physical information acquisition device 2 is fitted at the tip portion of the protrusion piece A1.

The protrusion piece A1 is an edge provided to protrude from the first attachment fixture 18 by 3 mm to 10 mm, and is provided to be perpendicular to the belt 3 when appropriately attached. The thickness thereof is at least 2 mm or more, and is provided at two locations of the upper end portion and the lower end portion of the attachment fixture body Q.

The fitting piece A2 is a component provided at the tip of the protrusion piece A1. The shape thereof is provided to be substantially the same as the shape of the attached portion 23 to be described later, so that it can be fitted with the attached portion 23, and is provided to have a portion at least wider than the width of the protrusion piece A1.

In the present embodiment, the fitting piece A2 is a cylindrical member having an outer diameter larger than the width of the attached portion 23, and a cross section has the same shape at any position.

The first insertion portion 182 is a portion that is integrally molded with the first attachment portion 181, through which the belt 3 is inserted, and an inserted portion is provided to have a rectangular parallelepiped shape having an opening in a substantially rectangular shape. The size of the rectangular shape is provided such that the height is at least larger than the width of the belt 3, and the width is at least larger than the thickness of the belt 3.

The structure of the second attachment fixture 19 is substantially the same as or completely the same as that of the first attachment fixture 18. As a result, the user can attach the physical information acquisition device 2 without worrying about an orientation of the attachment, and the handling performance can be improved.

The attached portion 23 is a hole portion provided from one end portion toward the other end portion in the lateral direction in the attachment fixture body Q. The shape thereof is provided corresponding to the shapes of the first attachment fixture 18 and the second attachment fixture 19 so that it can be attached to the first attachment fixture 18 and the second attachment fixture 19.

A depth of the hole is at least larger than the width of the fitting piece A2, and is preferably twice or more of the width. Further, it may penetrate the physical information acquisition device 2.

In the present embodiment, since the attached portion 23 is provided on the emission and entrance surface 22, the emission and entrance surface faces the user's body.

Further, the attached portion 23 is provided at intervals corresponding to a plurality of attachment portions provided in the attachment fixture body Q, thereby improving the holding performance.

The belt 3 is a belt-shaped member to be mounted to the waist part of the user, and includes a belt body 31 that is a body portion of the belt, and the attachment hole 32 that is a hole portion for penetrating the adjustment portion 15.

In the present embodiment, the belt 3 itself has a length adjustment function.

The belt body 31 is a belt-shaped member made of leather or synthetic resin, and has a length that can be wrapped around the user's waist. Hereinafter, the end portion of the belt body will be described as one end portion 31A and the other end portion 31B.

Hereinafter, an implementation method of the present invention will be described in detail with reference to Figs. 9 to 12. The present invention is implemented by a user having a purpose of attaching the physical information acquisition device 2 to his/her waist part. Further, the implementation method shown below is an example, and the implementation method is not limited thereto, and the order may be out of sequence.

### <<Implementation Method>>

First, the user attaches the attachment fixture 1 and the belt 3 to his/her waist.

In other words, the user attaches the first attachment fixture 18 and the second attachment fixture 19 to the belt by causing the belt body 31 to pass through the first insertion portion 182 and the second insertion portion 192. At this time, since both the attachment fixtures have the same structure and are bilaterally symmetrical in a front view, there is no need to worry about the order and orientation. At the same time, the user adjusts the length of the belt 3.

Next, the user attaches the physical information acquisition device 2 to the attachment fixture 1.

In other words, the user adjusts the distance between the first attachment portion 181 and the second attachment portion 191 to the distance between the attached portions 23, and as shown in Fig. 12, attaches the physical information acquisition device 2 by sliding it from top to bottom in a state where the positions of the first attachment portion 181 and the second attachment portion 191 are aligned with the positions of the attached portions 23.

At this time, a portion between the first attachment fixture 18 and the second attachment fixture 19 becomes a gap (the transmission portion 13), and the emission and entrance position of the emission and entrance surface 22 overlaps with the position of the transmission portion 13, and the portion can be fixed at the waist part, so that the user can appropriately mount the physical information acquisition device 2 that senses the body.

### <<Sixth Embodiment>>

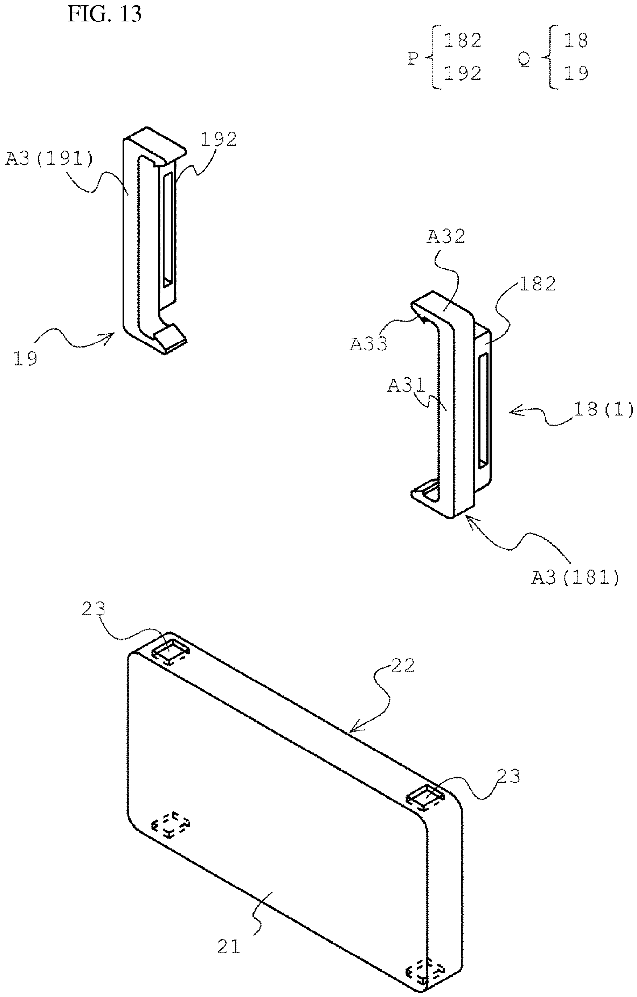

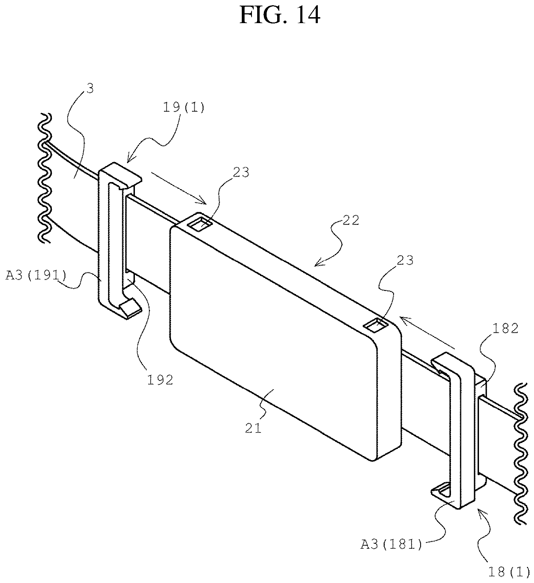

Hereinafter, an attachment fixture in a sixth embodiment of the present invention will be described with reference to Figs. 13 and 14. The description of the same components as those of each embodiment described above will be omitted.

The present embodiment is different from the tenth and fifth embodiments in a method of attaching the attachment fixture 1 and the physical information acquisition device 2.

The first attachment portion 181 is a hook body A3 made of plastic and having elasticity, and is integrally provided to be pasted to a surface of the first insertion portion 182 on which an opening surface is not provided.

The hook body A3 includes at least an attachment axis A31 that is longer than a surface to which the physical information acquisition device 2 is attached, a protrusion A32 that is provided to protrude perpendicularly to the attachment axis A31, and a claw A33 that is a hook portion provided inward at an end portion of the protrusion A32.

The direction in which the protrusion A32 protrudes may be parallel or perpendicular to an opening direction of the first insertion portion 182. Further, when a force is applied from the inside to the outside in the protrusion A32, the protrusion A is configured to elastically deform by at least the height of the claw A33.

The shape of the second attachment portion 191 is substantially the same as the structure of the first attachment portion 181, but is provided to be bilaterally symmetrical with the first attachment portion 181 so as to face each other when attached to the belt 3.

The attached portion 23 is a non-penetrating hole portion provided on a side surface of the physical information acquisition device 2. The attached portion 23 is provided to have a width wider than the claw A33 so that the claw A33 can be inserted, and is provided to have a depth at least deeper than the height of the claw A33.

Here, since the attached portion 23 is provided on a surface different from the emission and entrance surface 22, the emission and entrance surface 22 faces the user's body.

### <<Implementation Method>>

The user attaches the attachment fixture 1 to the belt 3 in the same manner as in the fifth embodiment.

Thereafter, the first attachment fixture 18 provided on the belt 3 is slid to align the positions of the attached portion 23 and the claw A33 of the physical information acquisition device 2, and the physical information acquisition device 2 is pushed and attached. Similarly, the second attachment fixture 19 is slid to align the positions of the attached portion 23 and the claw A33 of the physical information acquisition device 2, and the physical information acquisition device 2 is pushed and attached.

As a result, a portion between the first attachment fixture 18 and the second attachment fixture 19 becomes a gap (the transmission portion 13), and this portion can be fixed at the waist part, so that the user can appropriately mount the physical information acquisition device 2 that senses the body.

Further, in order to facilitate attachment and detachment of the claw A33, a member that can be gripped by the user may be provided on an upper surface of the protrusion A32.

### <<Seventh Embodiment>>

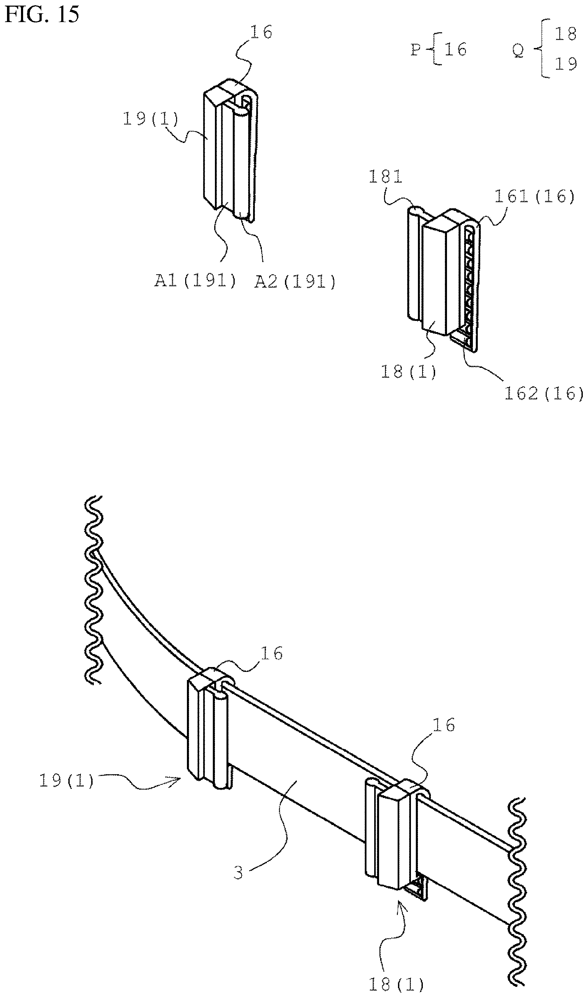

Hereinafter, the attachment fixture 1 according to a seventh embodiment of the present invention will be described in detail with reference to Fig. 15. The same components as those of each embodiment described above are denoted by the same reference numerals, and the description thereof will be omitted.

The attachment fixture 1 of the present embodiment is provided with a mechanism that attaches the physical information acquisition device 2 on the front surface thereof and attaches the physical information acquisition device 2 on the back surface thereof at the waist part of the user.

The attachment fixture 1 includes the attachment fixture body Q serving as the base portion of the attachment fixture 1, the first attachment portion 181 and the second attachment portion 191 to which the physical information acquisition device 2 is attached toward the user's body, the transmission portion 13 that transmits a measurement wave emitted from the physical information acquisition device 2, and the clip portion 16 that is attached by sandwiching the user's clothing (for example, trousers or a skirt) as the mounting portion P.

The clip portion 16 includes the clip body 161 that sandwiches the user's clothing with each of the attachment fixtures 1, and the friction portion 162 that acts as an anti-slip portion between the clothing and the attachment fixture body Q.

As shown in Fig. 15, the clip body 161 is a plate-shaped member provided to extend to the edge of the lower end with the edge of the upper end of each of the attachment fixtures 1 as a base portion.

The clip body 161 is provided at a position overlapping at least with each of the attachment fixtures 1 when viewed from the back, and is sandwiched on the user's clothing with each of the attachment fixtures 1.

The thickness of the clip body 161 is formed to gradually decrease from the base portion toward the tip portion, so that the clip body near the tip portion is easily deformed to facilitate attachment to clothing.

Further, the clip body 161 is configured to protrude outward at the base portion thereof, to approach the body portion of each of the attachment fixtures 1 as it moves downward, and to be substantially in contact with the body portion at lower end portion, thereby enabling firm attachment.

In the present embodiment, the friction portion 162 is provided with a plurality of irregularities on a surface in contact with the attachment fixture body Q, and holds the attachment fixture body Q by sandwiching clothing on the surface. On the other hand, the method of generating friction is not limited thereto, and a structure in which a rubber plate or the like is provided may be used.

### <<Implementation Method>>

A method of attaching the attachment fixture 1 according to the present embodiment to the user's clothing is as follows.

As shown in Fig. 15, the user inserts the upper end of his/her clothing such as trousers, a skirt, or a belt between the lower end of the clip body 161 and each of the attachment fixtures. As a result, the user can more easily mount the attachment fixture on the waist part.

Note that, in the present embodiment, since the belt is configured to be inserted between the clip portions 16, the belt may be inserted without being sandwiched.

### <<Eighth Embodiment>>

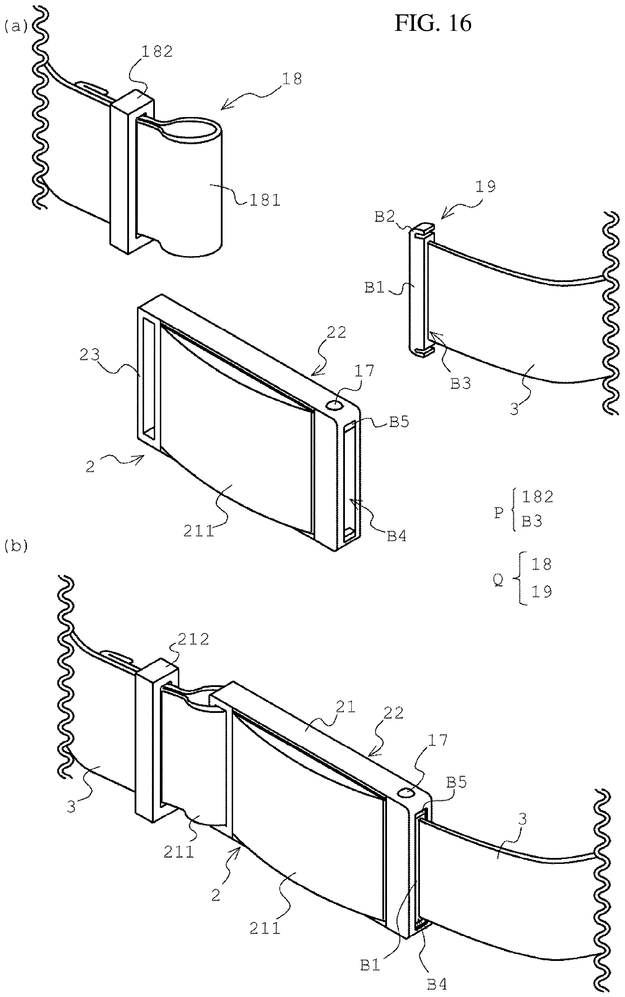

Hereinafter, the attachment fixture 1 according to an eighth embodiment of the present invention will be described in detail with reference to Fig. 16. The same components as those of each embodiment described above are denoted by the same reference numerals, and the description thereof will be omitted. Fig. 16(a) illustrates a state of the attachment fixture 1 before being attached, and Fig. 16(b) illustrates the attachment fixture 1 attached to the physical information acquisition device 2.

The attachment fixture 1 of the present embodiment includes the first attachment fixture 18 and the second attachment fixture 19. Each attachment fixture 1 includes an attachment portion for attaching the physical information acquisition device 2 and the mounting portion P that is mounted to the waist part of the user, respectively.

The first attachment fixture 18 includes the first attachment portion 181 in a ring shape and the first insertion portion 182 through which the belt 3 is inserted and that maintains the shape of the first attachment portion 181.

The first attachment portion 181 is a portion formed into a ring shape by causing the end portion of the belt 3 to pass through the first insertion portion 182 twice. The diameter of the ring is preferably 5 mm to 20 mm.

Note that, the first attachment portion 181 may be a ring-shaped member provided by attaching to first insertion portion 182.

The first insertion portion 182 is a loop member having a substantially rectangular parallelepiped shape through which the belt 3 is inserted, and a hole provided inside is at least larger than the width of the belt, and the width is larger than twice the thickness of the belt.

The second attachment fixture 19 includes an insertion portion B1 that functions as the second attachment portion 191 and is inserted into the attached portion 23 to be described later, and a connection portion B3 that has a detachable portion B2 for preventing unintentional removal and is connected to an end portion of the belt 3 as the mounting portion P to be mounted on the belt 3.

The insertion portion B1 is a substantially rectangular parallelepiped member having such rigidity that the insertion portion B1 cannot be bent by human force, and is provided to have substantially the same thickness as the attached portion 23 to be described later and a height smaller than that of the attached portion 23.

The detachable portion B2 is an elastic member provided to extend from both ends of the insertion portion B1, and a cut that generates a gap with the insertion portion B1 is provided from the direction of the connection portion B3. The width of the cut is provided to be at least larger than the height of a locking body B5 to be described later. Then, an interval between the detachable portions B2 is substantially the same as the height of an insertion port B4 to be described later, but is provided to be at least larger than the interval between locking bodies B5 to be described later, and when the detachable portion B2 is pushed inward, it can be deformed larger than the height of the locking body B5.

Further, the connection portion B3 is a hole portion that is fixed so as not to be removed in a state where the end portion of the belt 3 is inserted inside.

In the physical information acquisition device 2, a control button 211 of the physical information acquisition device 2 is provided such that it can be pressed against a surface facing the emission and entrance surface 22, and the attached portion 23 corresponding to each of the first attachment fixture 18 and the second attachment fixture 19 is provided.

Furthermore, since the two attached portions 23 are provided to face each other on two surfaces different from the emission and entrance surface 22, the emission and entrance surface 22 is exposed in a state of not being mounted, and the emission and entrance surface 22 is attached to face the user's body.

The attached portion 23 corresponding to the first attachment fixture 18 is a hole portion that is provided near the end portion of the physical information acquisition device 2 and penetrates the surface having the emission and entrance surface 22, and is provided to have a width at least larger than the thickness of the belt 3 and a height larger than the height of the belt 3.

The attached portion 23 corresponding to the second attachment fixture 19 includes the insertion port B4 through which the insertion portion B1 is inserted and attached, the locking body B5 that is provided on an inner peripheral surface of the insertion port B4 and cooperates with the detachable portion B2 to prevent the attachment fixture from falling off, and a pressing portion 17 that presses the detachable portion B2 in an attached state to remove the second attachment fixture 19.

The insertion port B4 is a hole portion provided on the side surface of the physical information acquisition device 2, and is provided such that the height thereof is higher than the sum of the heights of the insertion portion B1 and the two detachable portions B2, and by making the width thereof substantially the same as the width of the insertion portion B1, the insertion portion B1 can be inserted.

The locking body B5 is a protruding portion provided integrally with the insertion port B4 near an end surface of the inner peripheral surface of the insertion port B4, and the height thereof is substantially the same as the height of the detachable portion B2. One surface of the locking body B5 abuts on the end surface of the detachable portion B2 on the side of a connection portion when the second attachment fixture 19 is attached, thereby preventing unintentional removal.

The pressing portion 17 is a member provided on a side surface portion of the physical information acquisition device 2 and penetrates toward the insertion port B4. The pressing portion 17 can move up and down at least more than the height of the locking body B5 toward the center of the insertion port B4, and a part of the pressing portion 17 abuts on the detachable portion B2 in a state where the second attachment fixture 19 is attached.

Note that, the second attachment fixture 19 and the insertion port B4 may be attached by magnetic force by having different magnetism. Further, the second attachment fixture 19 and the insertion port B4 can be mounted by attaching a hook-and-loop fastener to the tip of the second attachment fixture 19 and a bottom surface of the insertion port B4, respectively.

Hereinafter, an implementation method of the present invention will be described in detail with reference to Fig. 16. The present invention is implemented by a user having a purpose of attaching the physical information acquisition device 2 to his/her waist part. Further, the implementation method shown below is an example, and the implementation method is not limited thereto, and the order may be out of sequence.

### <<Implementation Method>>

First, the user inserts the end portion of the belt 3 into the first insertion portion 182, inserts the end portion into the attached portion 23, and inserts the end portion into the first insertion portion 182 again to form the first attachment portion 181 in a ring shape. Then, the user wraps the belt 3 around the waist and adjusts the position of the first insertion portion 182 to adjust the size of the first attachment portion 181.

Next, the user attaches the insertion portion B1 of the second attachment fixture 19 to the insertion port B4. At this time, since the pushed detachable portion B2 is locked to the locking body B5, unintentional falling of the second attachment fixture 19 can be prevented.

As a result, the physical information acquisition device 2 can be attached to the waist part of the user.

The user removes the belt 3 by pressing the pressing portion 17.

In other words, when the pressing portion 17 is pressed, the detachable portion B2 is deformed, so that the interval between the detachable portions becomes smaller than the interval between the locking bodies B5, and the belt can be removed.

Embodiments of the attachment fixture according to the present invention also include the following modifications. However, the following modifications are examples of the configuration of the present invention similarly to each of the above embodiments, and the present invention is not limited to the following configurations.

### <<Modifications>>

As a modification according to the eighth embodiment of the present invention, as shown in Fig. 16, the components of the insertion portion B1 and the detachable portion B2 provided at the end portion of the belt 3 via the connection portion B3, the insertion port B4 and the locking body B5 provided along one edge of the acquisition device body 21, and the pressing portion 17 that enables removal of the insertion portion B1 and the insertion port B4 may be provided on both sides of the physical information acquisition device 2. In other words, the configuration of attachment of the insertion portion B1 and the insertion port B4 may be provided on two opposing edges so as to replace the attached portion 23 in Fig. 16. With such a configuration, the user can easily remove the physical information acquisition device 2 alone. Further, at this time, a configuration is preferable in which one insertion portion B1 is provided with an insertion portion through which the belt 3 can pass, and the length of the belt 3 can be adjusted according to Fig. 16.

In this modification, the pressing portion 17 may be provided on the side of the belt 3, that is, the insertion portion B1. At this time, the length of the insertion portion B1 in an insertion direction is longer than the depth of the insertion port B4, and when the insertion portion B1 is inserted into the insertion port B4 according to Fig. 16(b), the side of the belt 3 of the insertion portion B1 protrudes. This protruding length is slightly larger than the size of the pressing portion 17, and is preferably about 10% more to twice the length of the pressing portion 17 in the insertion direction. With such a configuration, the user can operate the pressing portion 17 and grip the insertion portion B1 at one time, and can remove the belt 3 from the physical information acquisition device 2 by a simple operation. Further, as shown in Fig. 16, the position of the pressing portion 17 may be provided on the surface in a thickness direction of the belt 3, the surface on the side of the emission and entrance surface 22, or the surface facing the emission and entrance surface, and the shape is limited to a circular shape, and may be provided in an oval shape, a rectangular shape with rounded corners, or the like.

The physical information acquisition device 2 according to each embodiment of the present invention may be provided with illumination. This illumination is preferably one with a diameter of 5 mm or less, such as an LED, which indicates the operation of the physical information acquisition device 2 and the remaining battery level.

The physical information acquisition device 2 according to each embodiment of the present invention may be provided with a sound collector. The sound collector acquires operation sound of the user's internal organs and sound around the user, so that information used for measurement of the user's health condition and position information and the like can be added.

### Reference Signs List

- 1: attachment fixture
- 11: attachment fixture body
- 111: holding portion
- 12: attachment portion
- 12A: lateral sliding attachment portion
- 12B: vertical sliding attachment portion
- 121: protrusion portion
- 122: fitting body
- 123: parallel piece
- 124: attachment portion body
- 125: insertion port
- 126: fastening portion
- 127: heat radiation hole
- 128: auxiliary transmission portion
- 13: transmission portion
- 14: insertion portion
- 141: adjustment portion body
- 142: coupling plate
- 15: adjustment portion
- 151: hole insertion portion
- 152: adjustment plate body
- 153: auxiliary plate
- 16: clip portion
- 161: clip body
- 162: friction portion
- 17: pressing portion
- 18: first attachment fixture
- 181: first attachment portion
- 182: first insertion portion
- 19: second attachment fixture
- 191: second attachment portion
- 192: second insertion portion
- A1: protrusion piece
- A2: fitting piece
- A3: hook body
- B1: insertion portion
- B2: detachable portion
- B3: connection portion
- B4: insertion port
- B5: locking body
- 2: physical information acquisition device
- 21: acquisition device body
- 211: control button
- 212: display unit
- 22: emission and entrance surface
- 23: attached portion
- 231: lateral attached portion
- 232: vertical attached portion
- 24: fitting portion
- 3: belt
- 31: belt body
- 32: attachment hole
- P: mounting portion
- Q: attachment fixture body

## Claims

1. An attachment fixture that can attach a physical information acquisition device that measures physical information of a user by emitting a measurement wave including a millimeter wave or a microwave from an emission and entrance surface toward the user to a belt, the attachment fixture comprising:
an attachment fixture body; a mounting portion that can be mounted to a waist part of the user; and an attachment portion to which the physical information acquisition device can be attached.

2. The attachment fixture according to claim 1, wherein
the mounting portion is provided with an insertion portion through which the belt is inserted, and further includes at least one of a second insertion portion or an adjustment portion that adjusts a length of the belt at a distance from the insertion portion.

3. The attachment fixture according to claim 1, wherein
the physical information acquisition device includes an attached portion that can be attached to the attachment portion.

4. The attachment fixture according to claim 3, wherein
the belt includes an insertion portion to be inserted into the attached portion at an end portion, and
the attached portion includes an insertion port into which the insertion portion is inserted.

5. The attachment fixture according to claim 4, wherein
the physical information acquisition device has a plurality of planes including the emission and entrance surface, and
the insertion port opens in one plane adjacent to the emission and entrance surface.

6. The attachment fixture according to claim 4, wherein
the attached portion is provided to face each other on two surfaces different from the emission and entrance surface.

7. The attachment fixture according to claim 2, wherein
the attachment fixture body includes a transmission portion that transmits the measurement wave between the insertion portion and the second insertion portion or the adjustment portion.

8. The attachment fixture according to claim 2, wherein
the adjustment portion is provided with a hole insertion portion that penetrates the belt.

9. The attachment fixture according to claim 3, wherein
the attachment portion is provided with a sliding portion that slides with the attached portion.

10. The attachment fixture according to claim 1, wherein
the attachment fixture body is a frame shape that leaves a central portion.

11. The attachment fixture according to claim 1, wherein
the attachment portion includes a protrusion portion that protrudes from the attachment fixture body.

12. The attachment fixture according to claim 1, wherein
the attachment portion includes a holding portion that holds an attachment state in which the physical information acquisition device is attached.

13. The attachment fixture according to claim 1, wherein
the attachment fixture body is provided to be separable.

14. The attachment fixture according to claim 1, wherein
the mounting portion includes a clip portion that can be mounted by sandwiching a member.

15. The attachment fixture according to claim 1, wherein
the attachment portion is provided with a lateral sliding attachment portion to which the physical information acquisition device is attached by sliding in a horizontal direction.

16. The attachment fixture according to claim 1, wherein
the attachment portion is provided with a vertical sliding attachment portion to which the physical information acquisition device is attached by sliding in a vertical direction.

17. The attachment fixture according to claim 3, wherein
the attachment portion is configured such that a distance from a portion mounted to the physical information acquisition device to the mounting portion is longer than a distance from the attached portion to the emission and entrance surface in a direction orthogonal to the emission and entrance surface.

18. A waist belt having the attachment fixture according to claim 1 or 2.

19. An attachment method for attaching a physical information acquisition device that measures physical information of a user by emitting a measurement wave including a millimeter wave or a microwave from an emission and entrance surface toward the user to a belt, the attachment method comprising:
attaching the physical information acquisition device such that the emission and entrance surface faces the user's body via an attachment fixture that includes an attachment fixture body, a mounting portion that can be mounted to a waist part of the user, and an attachment portion to which the physical information acquisition device can be attached.

20. A physical information acquisition device that measures physical information of a user by emitting a measurement wave including a millimeter wave or a microwave from an emission and entrance surface toward the user,
the physical information acquisition device being attachable to a belt by an attachment fixture that includes an attachment fixture body, a mounting portion that can be mounted to a waist part of the user, and an attachment portion to which the physical information acquisition device can be attached.
